Europäisches Patentamt

(19) European Patent Office

Office européen des brevets

(11) Publication number: **0 101 595**
**B1**

(12) **EUROPEAN PATENT SPECIFICATION**

(45) Date of publication of patent specification: **11.03.87**

(21) Application number: **83107783.9**

(22) Date of filing: **08.08.83**

(51) Int. Cl.⁴: **A 61 B 5/00**

(54) **Biological probes and methods of making same.**

(30) Priority: **09.08.82 US 406544**

(43) Date of publication of application:
**29.02.84 Bulletin 84/09**

(45) Publication of the grant of the patent:
**11.03.87 Bulletin 87/11**

(84) Designated Contracting States:
**AT BE CH DE FR GB IT LI LU NL SE**

(56) References cited:
**DE-A-2 003 138**
**DE-A-2 501 594**
**US-A-3 411 507**
**US-A-3 951 136**
**US-A-4 176 660**

(73) Proprietor: **VITAL SIGNS, INC.**
**1 Madison Street**
**East Rutherford New Jersey 07073 (US)**

(72) Inventor: **McGinnis, Gerald E.**
**131 Kelvington Dr.**
**Monroeville Pennsylvania 15146 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

Courier Press, Leamington Spa, England.

## Description

### Background of the Invention

The present invention relates to biological probes and to methods of making such probes. Probes according to the present invention are particularly useful as endoesophageal probes for insertion into the esophagus of a human subject to monitor physiological parameters of the subject.

Various probes are widely utilized in the biological and medical arts for monitoring conditions or applying treatments within a living organism. Such probes generally include an elongated structural member and sensing transducers, fluid administration ports or both adjacent the distal end of such member. The transducers and ports may be positioned at the desired locations within the organism by advancing the structural member, distal end first, into the organism. Ordinarily, the structural member includes a sheath or exterior portion defining a smooth exterior surface on the distal region of the probe. The sheath may be formed primarily from a soft plastic material to permit the probe to bend and follow natural body passageways during insertion.

One example of such a biological probe is the multiple purpose esophageal probe described in U.S. Patent No. 3,951,136, issued April 20, 1976 to Terence D. Wall. Some embodiments of the probe disclosed in the Wall patent include an elongated tubular structural member, a temperature sensing transducer, and a thin flexible diaphragm mounted at the distal end of the tube, the diaphragm surrounding the transducer and closing off the distal end of the tube. The probe may be inserted into the esophagus of a patient so as to position the temperature sensor within the body of the patient at a location near the heart for monitoring so-called "core body temperature". The diaphragm shields the temperature transducer from the gastric juices present in the esophagus, but permits effective transmission of body sounds into the tube. Thus, the Wall probe also serves as an intra-esophageal stethoscope, body sounds being transmitted through the bore of the tube so that they may be monitored through an ear piece connected to the proximal end.

One or more electrically conductive electrodes may be provided on a biological probe. For example, the aforementioned Wall patent discloses a probe in which the sheath or exterior surface portion is formed primarily from an electrically insulating plastic material, a pair of metallic electrodes being fastened to the plastic material in the distal region of the probe. These electrodes may be rigid, plated brass buttons having smooth dome-like portions protruding from the plastic portion of the sheath so that the electrodes and plastic portion cooperatively define a relatively smooth exterior surface. The electrodes are provided with electrical leads or conductors extending to the proximal end of the probe, so that the electrodes may be utilized to detect the electrocardiogram voltages present in the esophageal region of the body. DE—A—2,003,138 also discloses a probe having electrodes for monitoring electrocardiogram voltages. US—A—3,411,507 discloses a probe having a single electrode adjacent its distal end, such probe being used to apply a stimulating electrical voltage to the interior of the digestive tract.

US—A—4,176,660 discloses a similar probe wherein the electrical conductors leading to the electrodes are composed of a blend material including polymeric material and an electrical conductive non-polymeric material embedded in the insulating portion of the sheath and the electrodes are formed by carbon impregnated heat shrinkable vinyl collars which are slipped over the tube and heat shrunk thereupon. Interconnection are made to one of the conductors by scraping away a portion of the insulating portion of the sheath prior to heat shrinking. Such heat shrunk collar, however, do not form a reliable electrical connection with the conductors and form producing portions on the sheath of the tube.

There is need for improvement in the structure of probes having electrodes which would render such probes less likely to cause trauma or mechanical injury to the tissues of the organism during use. There is also a need for improvement in the reliability of the electrical connection between the electrodes and the associated electrical conductors, and reduction in the bulk of such connection. Further, there are needs for simplification of the processes used in manufacture of the probes, and for improvement in the inherent reliability of the processes to reduce the proportion of defective probes produced.

The invention as claimed in claims 1 and 17 solves the problem of how to design a biological probe for insertion into the body of a living organism by which the danger of a trauma or mechanical injury of the tissues of the organism is reduced and the reliability of the electrical connection between the electrodes and the associated electrical conductors is improved.

Claims 8 and 21 are directed to methods of producing such a biological probe.

The present invention incorporates the realization that the tissue trauma encountered with probes utilized heretofore has been caused at least in part by movement of normally contiguous portions of the probe surface relative to one another. Such relative motion may occur if the sheath defining the exterior surface of the probe incorporates soft, flexible material. As the probe deflects during use, the soft material may deform while the immediately adjacent portions of the electrode do not deform. Thus, the edges of the electrodes may be exposed and may cut or tear the surrounding tissue. Also, the surrounding tissue may be pinched between the electrode surface and the surrounding portions of the probe surface as such surfaces move towards and away from one another.

The insulating and conductive portions cooperatively define a smooth exterior surface, and relative motion between normally contiguous portions of such exterior surface deformation of the sheath is prevented. When the sheath deforms the conductive, coated electrode portions will deform with the surrounding insulating portions, thus preventing any relative movement between normally contiguous portions of the exterior surface.

Preferably, the conductive material has elastic properties close to those of the contiguous insulating material. This arrangement reduces the stresses imposed upon the bond during flexure. The insulating material may be a polymeric material and the conductive material constituting the electrode, whether in the form of a coating or in the form of a mass, may be formed from a blend material including a polymeric material and an electrically conductive non-polymeric material. For example, the insulating portion of the sheath may be composed of plasticized polyvinyl chloride and the conductive portion may be composed of a blend comprising plasticized polyvinyl chloride and carbon black.

A probe according to the present invention also includes means for conducting an electrical signal between the electrode portion of the sheath and a location accessible during use of the probe. Such means may include an electrical conductor embedded in the insulating portion of the sheath. The conductor may be formed from a blend material including a polymeric material and an electrically conductive non-polymeric material. The conductor may be fused with the insulating material of the sheath for physical support and the conductor may also be fused with the electrode to provide a secure and reliable electrical connection therebetween.

In one method of making a probe according to the present invention, the electrode may be formed by depositing a liquid, curable material onto the external surface of a sheath portion composed of an electrically insulating material so that the liquid material wets the insulating material and flows to a contour blending smoothly with the external surface of the insulating portion. The liquid material is cured to a solid, electrically conductive form so as to form a solid electrode on the external surface of the insulating portion, thereby forming a unitary, composite sheath including insulating and conductive portions. Because the contour of the conductive portion or electrode is formed by the flow of the liquid material under the influence of its inherent surface and interfacial tensions, the difficulties encountered in fitting a pre-formed electrode to the insulating portion of the sheath to provide a smooth surface are eliminated.

A portion of an embedded conductor may be exposed prior to application of the liquid material, and the liquid material may be applied so that it contacts the exposed portion of the conductor. If the carrier in the liquid material is a solvent for a polymer incorporated in the conductor, the electrode or conductive portion of the sheath will be fused to the conductor upon curing. Thus, each electrode may be formed, bonded to the insulating portion of the sheath and connected to the conductor in a single operation.

In a manufacturing process according to another aspect of the present invention, a mass of electrically conductive material is juxtaposed with a sheath portion composed of insulating material so as to form a composite sheath, an exposed surface of the conductive mass forming a part of the exterior surface of the composite sheath. A bond is formed at the interface between the conductive mass and the insulating portion, such bond extending to the extremity of the interface at the exterior surface of the composite sheath. Such bond may be made by forming and then curing a liquid phase at the interface.

Preferably, a bead or pool of the liquid phase is formed on the exterior surface of the composite sheath adjacent the interface between the conductive mass and the insulating portion. The liquid in such bead flows to a smooth contour extending between the exposed surface of the conductive mass and the adjacent exposed surface of the insulating portion, and the liquid phase is cured in such a fashion as to retain the smooth contour. This method permits formation of a composite sheath having a smooth exterior surface without the need for special care in fitting the conductive and insulating portions to one another.

The foregoing and other objects, features and advantages of the present invention will be more readily apparent from the detailed description of preferred embodiments set forth below taken in conjunction with the accompanying drawings.

Brief Description of the Drawings

Fig. 1 is a schematic perspective view of a probe according to a first embodiment of the present invention, a portion of the probe being removed for clarity of illustration.

Fig. 2 is a fragmentary sectional view taken along line 2—2 in Fig. 1.

Fig. 3 is a fragmentary sectional view on an enlarged scale of the area indicated at 3 in Fig. 2.

Fig. 4 is a schematic block diagram of a manufacturing process utilized in making the probe illustrated in Figs. 1—3.

Fig. 5 is a schematic electrical circuit diagram of a probe according to a second embodiment of the present invention, in conjunction with certain electrical signal processing and monitoring devices.

Fig. 6 is a schematic, perspective cutaway view depicting portions of a probe according to a third embodiment of the present invention.

Fig. 7 is a fragmentary sectional view on an enlarged scale of the area indicated at 7 in Fig. 6.

Fig. 8 is a view similar to Fig. 7 but depicting a portion of a probe according to a fourth embodiment of the present invention.

Detailed Description of the Preferred Embodiments

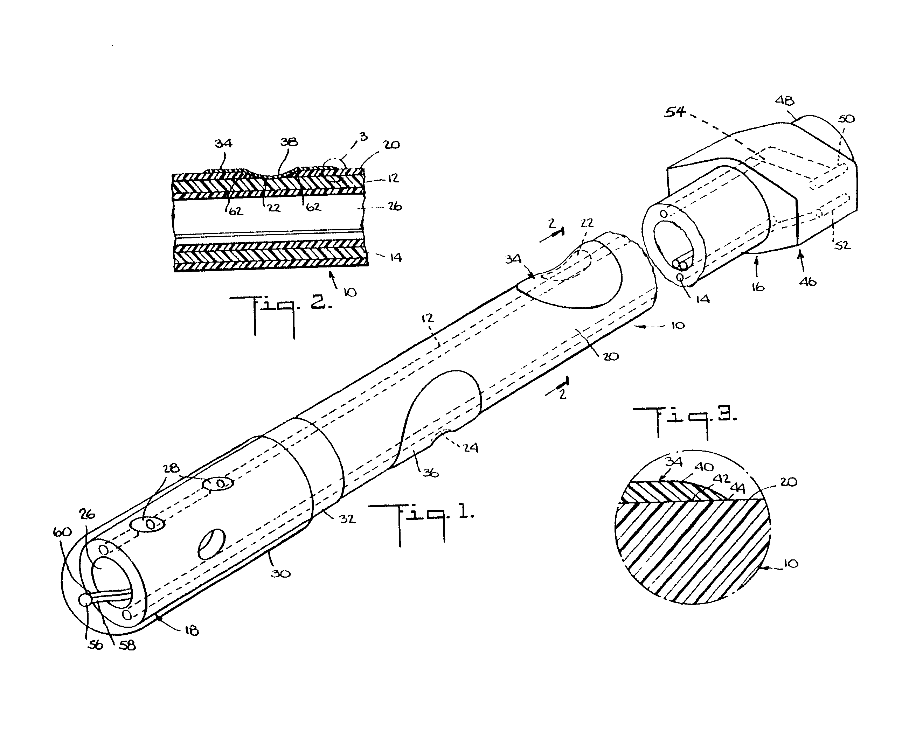

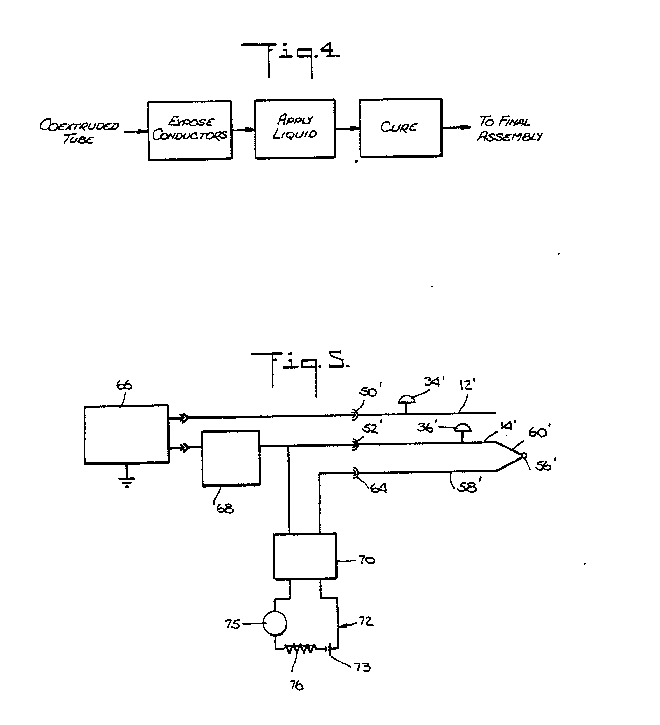

As illustrated in Figs. 1 and 2, a probe according to a first embodiment of the present invention includes an elongated tube 10 of electrically insulating plasticized polyvinyl chloride ("PVC"). The tube is about 22 inches (56 cm) long and has an outside diameter of about 0.24 inches (6 mm). Tube 10 has a pair of electrical conductors 12 and 14 embedded in its wall at diametrically opposed locations, the conductors extending the entire length of the tube from its proximal end 16 to its distal end 18. Conductors 12 and 14 are composed of a blend material including PVC and carbon black. The conductors constitute integral parts of the tube wall, the blend material of the conductors being fused with the remainder of the tube wall. Tube 10 has a cylindrical exterior surface 20 and a pair of depressions 22 and 24 extending inwardly from such exterior surface at axially spaced, diametrically opposed locations in the distal region of the tube. Depression 22 is about $3\frac{7}{8}$ inches (9.8 cm) from the distal end of the tube, and is aligned with conductor 12. Depression 24 is about $2\frac{1}{2}$ inches (6.4 cm) from the distal end of the tube and is aligned with conductor 14. As best seen in Fig. 2, depression 22 extends through the outermost region of insulating material in the tube wall to conductor 12, but the depression does not extend entirely through the wall of the tube to the interior bore 26. Depression 24 likewise extends only partially through the wall of the tube to conductor 14.

A plurality of apertures 28 extend entirely through the wall of tube 10 adjacent the distal end of the tube. A thin, flexible bulb-shaped membrane 30 having a collar 32 at its proximal end is affixed to the distal end of the tube, collar 32 being sealingly bonded to the surface of the tube proximally of apertures 28 so that membrane 30 overlies the apertures and the open distal end of bore 26.

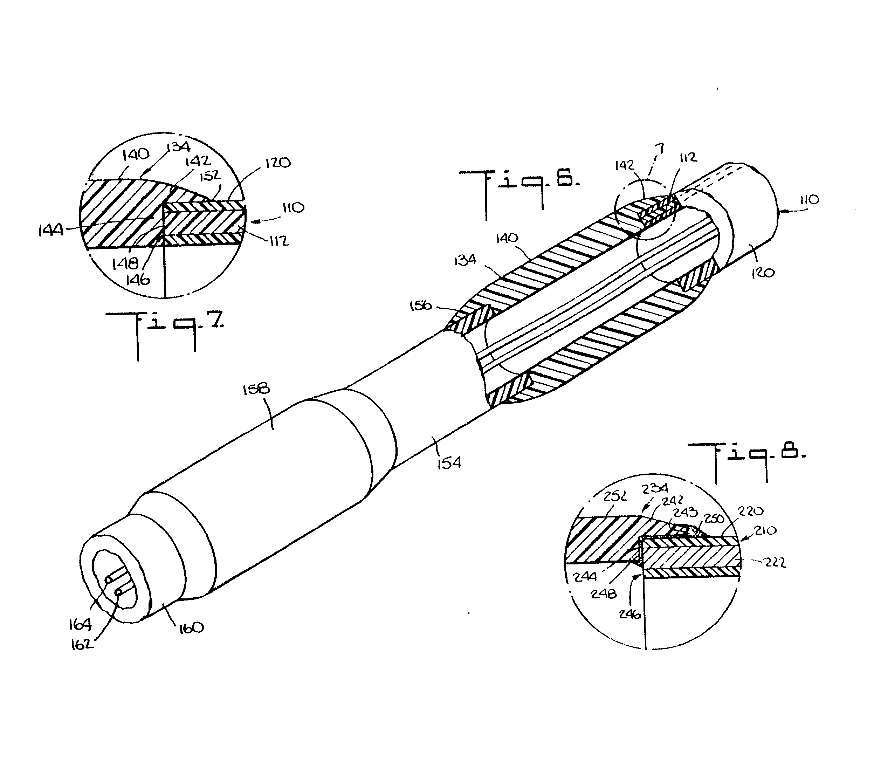

The sheath of the probe also includes two electrically conductive portions or electrodes 34 and 36. Electrode 34 consists of a thin film of a blend material including PVC and carbon black, such film extending over a portion of the cylindrical surface 20 of tube 10 in the vicinity of depression 22, the film being bonded to the underlying insulating material of the tube. Such film also extends into depression 22 so that a portion 38 of the film disposed within the depression is contiguous with conductor 12, such contiguous portions of the electrode and the conductor being fused to one another. Film 34 is about .010 inches (0.25 mm) thick. As best illustrated in Fig. 3, the exposed surface 40 of film 34 merges with the exterior surface 20 of tube 10 in such a fashion that the two surfaces cooperatively constitute a smooth, unitary exposed surface.

As the term "smooth" is utilized in this disclosure, a surface of a solid may be considered "smooth" if it is devoid of any sharp projection more than .015 inch (0.38 mm) in height or outward extent from an immediately adjacent portion of the surface. A projection may be considered "sharp" if it has, at its outermost extremity, an edge or corner of less than .005 inch (.013 mm) radius and less than 120° included angle (as measured through the solid at the edge or corner). Two surfaces may be said to merge "smoothly" with one another if the juncture between such surfaces is devoid of any such sharp projection. Although "smooth" surfaces meeting the above criteria can be employed in contact with tissues such as those lining the human esophagus, still smoother surfaces meeting closer criteria (lesser maximum projection height and greater minimum radii) are most desirable to further minimize tissue trauma.

The bond between the conductive film 34 and the insulating tube 10 extends along the interface 42 therebetween to the outer extremity 44 of such interface at the exterior surface of the sheath, *i.e.,* the extremity of the interface at the juncture between surface 40 of the film and surface 20 of the tube.

The blend material of film 34 and the contiguous insulating material of tube 10 are both soft materials. As used in this disclosure, the term "soft material" means a material having a Young's modulus (tensile elastic modulus) of less than about $3 \times 10^5$ pounds per square inch or psi ($2.07 \times 10^9$ Nt/m²). Because the elastic properties of the film and the underlying tube material are comparable to one another, there is no such discontinuous difference in elastic properties across the interface between the film and the underlying insulating material.

A body devoid of such discontinuities may be characterized as an "elastically continuous" body. In this context, a discontinuity means a gradation in Young's modulus of more than about $3 \times 10^5$ psi ($2.07 \times 10^9$ Nt/m²) over a distance of less than about .015 inch (0.38 mm). Two adjoining bodies constituting parts of a larger body may be characterized as being elastically continuous with one another if there is no such discontinuity at the juncture therebetween. For example, because the film defining electrode 34 and the contiguous insulating portion of tube 10 are both formed from soft materials having Young's moduli less than $3 \times 10^5$ psi ($2.07 \times 10^9$ Nt/m²), the difference between the Young's moduli of these bodies must be less than $3 \times 10^5$ psi ($2.07 \times 10^9$ Nt/m²) and they are therefore elastically continuous with one another. Such elastic continuity between electrode 34 and the underlying tube tends to moderate stresses at interface 42 during deformation of the probe. As will be appreciated, such stresses can be minimized by reducing the differences in physical properties between the film material and the tube insulating material to a minimum. It is especially desirable to provide elastic continuity between the film and the insulating tube if the tube is formed from a very soft material such as a highly plasticized polyvinyl chloride polymer of between about 50 and about 80 Shore A Durometer. Such materials generally have Young's moduli of about $5 \times 10^4$ to about

$1 \times 10^3$ psi (about $3.45 \times 10^8$ Nt/m² to about $6.89 \times 10^6$ Nt/m²). The material of the film should preferably be selected so that its elongation at break — the degree to which it can be stretched without breaking — is at least about one fourth the elongation at break of the underlying tube material. Ordinarily, if the elongation at break of the film material is equal to or greater than the elongation at break of the tube material, the film will not break during deformation of the probe unless such deformation is so severe as to break the tube itself.

The film constituting electrode 34 is also elastically continuous with the conductor 12. This tends to moderate stresses at the points of fusion between the conductor and the film during flexure of the probe. The second conductive portion or electrode 36 is substantially similar to electrode 34, except that electrode 36 is fused to conductor 14 and extends over the surface of the tube in the vicinity of depression 24.

A connector block 46 composed of a rigid plastic is bonded to the proximal end 16 of tube 10. The connector block has a hollow fitting 48 communicating with the interior bore 26 of the tube, and the fitting has a Luer taper suitable for connection to a stethoscope assembly (not shown). Two metallic female electrical pin connector receptacles 50 and 52 are embedded in block 46, each such receptacle having an opening at the surface of block 46. Receptacle 50 has a metallic extension 54 extending through block 46 and into the wall of tube 10 to conductor 12. Receptacle 50 is thus electrically connected to conductor 12 and to electrode 34. The other receptacle 52 is similarly electrically connected to conductor 14 and thus to electrode 36. Although each electrode is electrically connected to one of the receptacles, each electrode is electrically isolated from the other electrode and from the conductor and receptacle associated with such other electrode.

A temperature transducer in the form of a thermistor 56 is mounted at the distal end of the probe, within membrane 30. Thermistor 56 is physically supported by two insulated electrical leads 58 and 60 which protrude from the distal end 18 of tube 10. These leads extend through interior bore 26 to the proximal end of tube 10, each such lead being electrically connected to a further electrical pin receptacle (not shown) at connector block 46, thermistor 56 being electrically connected by way of the leads to such further receptacles, but electrically isolated from the electrodes and from the conductors and receptacles associated therewith.

In use, the distal portion of the probe may be inserted into the esophagus of a human subject until the distal end of the probe is disposed in the lower portion of the esophagus, thus positioning electrodes 34 and 36, membrane 30 and temperature transducer 56 in the so-called "core" region of the body. A stethoscope ear piece may be connected to fitting 48 so that the physician may listen to the patient's heart sounds, such sounds being efficiently transmitted through thin membrane 30 and into the interior bore 26 of the tube through the open distal end of such bore and through apertures 28, the sounds being transmitted via bore 26 to the stethoscope ear piece. An appropriate instrument may be connected to the thermistor via leads 58 and 60 and the receptacles associated therewith to monitor the electrical resistance of thermistor 56 and thus monitor the temperature of the core body region surrounding the thermistor.

Electrodes 34 and 36 bear on the interior wall of the esophagus at spaced locations along the esophagus, each electrode being intimately electrically connected to the tissue of the esophageal wall by contact with such tissue. Thus, the electrical potential or voltage at each electrode will vary in accordance with the electrical potential of the esophageal tissue immediately adjacent thereto. These potentials vary in accordance with the electrical activity of the patient's heart. A conventional electrocardiograph may be connected to receptacles 50 and 52 to monitor the pattern of variation of these voltages.

As the probe is inserted into the esophagus, the exterior surface of the sheath will wipe across the interior surface of the esophagus and the surfaces of associated anatomical structures. Because the exterior surface of the probe sheath, defined by the outermost layer of tube 10 and by electrodes 34 and 36, is a continuous, smooth surface, the probe will not tend to tear or abrade the tissues constituting these body surfaces. As the esophagus and associated anatomical passageways are normally curved, the probe will bend during insertion and removal. The probe may also bend, and may move relative to the adjacent tissues, upon movement of the patient. When the probe bends and the probe sheath deforms to accommodate such bending, those portions of the probe defining its exterior surface in the vicinity of the electrodes — the outer skin of tube 10 and the films constituting electrodes 34 and 36 — will deform as a unit. Because the bonds between the electrodes and the underlying insulating material of the tube extend to the extremities of the electrodes, the peripheral portions of the electrodes will not peel away from the surrounding exposed portions of the tube. Thus, although the surfaces of the electrodes and the tube will deform, contiguous portions of the electrode surface and the tube surface at the peripheries of the electrodes will not move relative to one another. The exterior surface of the probe in the vicinity of the electrodes will thus remain smooth and continuous during bending of the probe in normal use.

The probe described above with reference to Figs. 1—3 may be made by a process such as that schematically illustrated in Fig. 4. The process starts with a preformed tube having the conductors embedded in its walls. Such a tube may be formed by conventional coextrusion processes, the blend material forming the conductors being extruded simultaneously with the insulating material forming the remainder of the tube. In the probe-manufacturing process, depressions are formed in the exterior surface of the coextruded tube to expose the conductors, each such depression being in the form of a broad shallow U-shaped notch in the tube wall, the notch having ramp-like surfaces disposed at acute angles to the axis of the tube. The depression 22 illustrated in Fig. 2 has such a configuration. A depression in this form may be made by notching the tube with a cylindrical punch having a radius greater than the desired depth of the depression. The axis of the punch is maintained perpendicular to the axis of the tube but displaced therefrom, and the punch is moved along its axis so that an edge region of the punch engages the tube. The tube may be gripped between a pair of opposed pressure pads during this process to accurately position the tube with respect to the punch axis. Alternatively, each depression may be formed by first bending the tube and then slicing the portion of the tube at the outside of the bend with a razor blade, moving the razor blade generally along a chord of the bend.

After the depressions have been formed, a curable liquid material is applied to the tube in the vicinity of the depressions so that the liquid material at each depression contacts the conductors exposed in such depression. The liquid material must be a material capable of forming an electrically conductive solid of suitable physical properties upon curing. Suitable liquid materials may incorporate a polymeric material and an electrically conductive non-polymeric material such as carbon black dispersed in a liquid carrier. The liquid carrier may be volatile, so that the material can be cured by evaporation of the carrier.

Such a volatile liquid carrier should preferably be a solvent for the polymer incorporated in the liquid material and for the polymers incorporated in the insulating material of the tube and in the blend material of the conductor. If such a solvent is utilized, the films produced upon evaporation of the solvent will be fused with the conductors and with the insulating material of the tube. If polyvinyl chloride polymers are incorporated in the liquid material, the insulating material and the blend material of the conductors, tetrahydrofuran may be utilized as the liquid carrier. Generally, the use of similar polymers in the various materials facilitates the choice of a single liquid carrier which is a solvent for all of the polymers. If the polymeric material requires plasticizers or other additives to impart suitable physical properties after curing, such additives may also be dissolved in the liquid carrier.

Other curable liquids may be utilized, such as liquids comprising a conductive material dispersed in a polymerizable urethane or epoxy liquid phase.

The liquid material may be applied to the tube by brushing, spraying or dipping so as to form a coating of the desired thickness. Ordinarily, the liquid material will be applied over those portions of the tube surface surrounding each depression and some liquid material will also be applied within each depression. Under the influence of its own surface tension and its interfacial tensions with the underlying materials of the tube, the liquid material flows to a liquid film having a smooth continuous exposed surface merging smoothly with the surrounding exposed insulating surface of the tube. If the liquid film is cured to a solid state without disturbing its surface, the final solid film constituting the electrode will have a correspondingly smooth surface which will also merge smoothly with the tube surface. If the liquid film is of a type which can be cured by application of heat, the curing step may be performed by heating the cured tube without touching the liquid film with any solid object. For example, the coated tube may be placed in a conventional oven or subjected to infrared radiation. Each film may be built up by multiple coating steps, with each coat being cured prior to application of the next coat.

A film applied as described above tends to mask minor imperfections on the tube surface at the depressions. For example, if the depression forming step leaves sharp edges 62 (Fig. 2) at the borders of each depression, such edges will be masked by the overlying film. Application of the electrodes to the tube by the liquid film process described above thus provides important advantages in the finished product including a secure bond of the electrode to the underlying insulating material and a secure bond between the electrode and the conductor which provides a secure electrical connection between these elements. The liquid film coating process produces an especially smooth juncture between the exposed surfaces of the electrode and the tube. Moreover, the process is inherently reliable and does not require close fitting of mating parts or special care in control of part dimensions. So long as the depressions do not extend through the wall of the tube to the interior bore, there is no possibility of leakage through the tube wall at the depressions during use of the finished product. Because the same liquid application and curing steps which produce the electrodes also produce the electrical interconnection between the electrodes and the conductors, and the bonds between the electrodes and the tube, separate fastening and electrical interconnection steps may be eliminated.

After the electrode films have been cured, the other components of the probe may be assembled to the tube. The apertures at the distal end of the tube may be formed either before or after application of the electrodes.

A probe according to a second embodiment of the present invention is illustrated in Fig. 5. This probe has structural elements similar to those of the probe described above with reference to Figs. 1—3, including electrodes 34' and 36' connected to conductors 12' and 14' embedded in the wall of the tube (not shown). The embedded conductors are provided with receptacles 50' and 52' respectively, such receptacles being mounted in a connector block (not shown) at the proximal end of the tube. This probe also incorporates a temperature sensing thermistor 56' at the distal end of the probe. Thermistor 56' is provided with a lead 58' extending through the interior bore of the probe tube to a further receptacle 64 at the connector block. However, the other lead 60' of thermistor 56' does not extend through the interior bore of the tube. Rather, lead 60' is a short, stub lead extending only to the distal end of embedded conductor 14'. Thus, embedded conductor 14' serves both as a conductor for the electrocardiogram voltage from electrode 36' and as a lead for thermistor 56'.

In use, receptacle 50 is directly connected to an electrocardiograph 66. Receptacle 52' is connected to the electrocardiograph by way of a high pass electrical filter 68. The high pass filter may be a conventional resistor and capacitor network arranged to transmit high frequency or rapidly varying electrical voltages but block transmission of invariant or low frequency, slowly varying voltages. For example, the high pass filter may be arranged to block transmission of voltages varying at frequencies of less than about 10 cycles per minute (0.16 Hz). Receptacle 52' is also connected by way of a low pass electrical filter 70 to a resistance monitoring instrument or ohmmeter 72, receptacle 64 being connected through a separate branch of the low pass filter to such instrument. Low pass filter 70 may also include resistor and capacitor networks, but the low pass filter is arranged to block transmission of rapidly varying voltages and permit transmission of slowly varying or invariant voltages, such as those varying more slowly than about 10 cycles per minute (0.16 Hz).

In its simplest form, ohmmeter 72 may consist of a battery 73, galvanometer 75 and constant resistance 76 in series with one another, such elements being connected through the filter in series with thermistor 56'. The voltage imposed by battery 73 on this series loop is substantially constant. Thus, the sum of the voltage across thermistor 56' and the voltage across resistance 76 is also substantially constant, such sum being equal to the voltage imposed by the battery. As the resistance of thermistor 56' increases, the current flowing in the series loop will decrease, the voltage across constant resistance 76 will also decrease and the voltage across thermistor 56' — the voltage imposed across the thermistor by the monitoring instrument — will increase.

The electrocardiogram voltages generated by the patient's heart and of interest to the physician are rapidly varying voltages having frequencies equal to or greater than the patient's pulse frequency, *i.e.* above about 30 cycles per minute (0.5 Hz). The electrical resistance of the thermistor varies with the temperature of the patient's body, and the voltage imposed across the thermistor by resistance monitoring device 72 will vary correspondingly. Such temperature variations and the corresponding voltage variations ordinarily will have frequencies less than 10 cycles per minute (0.16 Hz). Thus, the high pass filter 68 will effectively block transmission to the electrocardiograph of voltage variations at receptacle 52' caused by relatively low frequency variations in the resistance of thermistor 56' while effectively transmitting to electrocardiograph 66 the relatively high frequency voltages detected by electrode 36'. Conversely, low pass filter 70 will effectively block transmission of the high frequency electrocardiogram voltages to monitoring device 72 while permitting such monitoring device to sense the relatively low frequency variations in the resistance of thermistor 56' which indicate changes in body temperature.

Use of the embedded conductor as a substitute for one of the thermistor leads in this fashion saves some of the space within the interior bore of the tube ordinarily occupied by the omitted thermistor lead. This is advantageous in that it provides more clear area within the tube and thus promotes better transmission of heart sounds along the length of the tube. Moreover, the cost of the omitted lead and the cost of the separate receptacle which would otherwise be associated with such lead are eliminated. Of course, in this embodiment, the embedded conductor 14' extends to the vicinity of the thermistor and thus extends substantially the full length of the tube. However, this does not impose any added cost if the embedded conductor is formed by coextrusion of the tube insulating material and the conductor material. Such a process ordinarily produces embedded conductors extending the full length of the tube whether or not the full conductor length is desired.

Portions of a probe in accordance with a third embodiment of the present invention are depicted in Figs. 6 and 7. This probe includes a first tube 110 of insulating material, such tube having a conductor 112 embedded therein. Tube 110 extends to the proximal end of the probe. A first electrode formed from a mass of electrically conductive material in the form of a hollow tube 134 is mounted at the distal end of tube 110. A thin annular portion 142 of tube 134 at the proximal end of such tube surrounds the distal extremity of tube 110, annular portion 142 being disposed radially outwardly of tube 110. The radially inward face of the annular portion confronts the exterior surface 120 of tube 110. The smooth exterior surface 140 of tube 134 tapers radially inwardly toward the proximal extremity or edge of annular portion 142, so that surfaces 140 and 120 merge smoothly with one another. Conductive tube 134 has an abutment wall 144 extending radially inwardly from annular portion 142, such abutment wall confronting the distal end face 146 of tube 110 and confronting the distal end face 148 of conductor 112. The confronting surfaces of these tubes 134 and 110 are bonded to one another, such bond extending along the interface between tubes 134 and 110 to the outer extremity 152 of such interface at the exterior surface of the probe, such bond also extending around the entire circumference of tube 110. The abutment wall 144 is bonded to face 148 of conductor 112 and electrically connected thereto.

A further tube 154 formed entirely from an insulating material is connected to the distal end of conductive tube 134. The joint between the conductive tube and the further insulating tube 154 is similar to the joint between the conductive tube and tube 110, described above. Thus, the bond between these tubes extends to the outer extremity of the interface between them, but there is no electrical connection at the interface of tubes 134 and 154. A second electrode 158 is provided in the form of a second tube of conductive material mounted to the distal end of insulating tube 154 and another tube 160 composed entirely of insulating material is connected to the distal end of tube 158, the junctures between these tubes being similar to the junctures described above. A sound transmitting membrane and a thermistor (not shown) are mounted at the distal end of tube 160. The thermistor is provided with two leads 162 and 164 which extend through the interior bores of the aforementioned tubes to the proximal end of the probe. A portion of lead 162 within conductive tube 158 is stripped of its insulation and embedded in the conductive material of this tube at a location remote from the exterior surface of the tube. Thus, lead 162 is electrically connected both to the thermistor and to tube 158. Embedded conductor 112 and leads 162 and 164 are electrically connected to appropriate receptacles (not shown) at the proximal end of the tube. The probe may be connected to an electrocardiograph and a resistance monitoring instrument through high pass and low pass filters in an arrangement similar to that described above with reference to Fig. 5.

The exterior surfaces of the conductive tubes or electrodes 140 and 158, and of the insulating tubes 110, 154 and 160 cooperatively constitute a continuous, smooth surface on the exterior of the probe in the vicinity of the electrodes. The bonds between the conductive and insulating tubes prevent movement of contiguous portions of this surface upon deformation of the probe such as may be encountered in normal use. All of the tubes are preferably formed from soft materials incorporating polymers similar to one another, i.e., polymers of the same monomer, and the insulating and conductive tubes are preferably elastically continuous with one another so as to minimize stresses at the bonds. The taper of annular portion 142 also tends to minimize stresses at the portions of the bond adjacent outer extremity 152. Similar tapers at the other extremities of the conductive tubes likewise tend to minimize stresses at the bonds between such tubes and the adjacent insulating tubes.

The electrodes of the probe illustrated in Figs. 6 and 7 may be separately formed, as by injection molding and then joined with the tubes by applying a solvent at the interfaces between the electrodes and adjoining tubes. Alternatively, they may be formed by coating the electrodes from a curable liquid phase, as by positioning insulating tubes 110, 154 and 160 on a mandrel extending through the internal bores of these tubes, then applying a curable, viscous liquid material to the mandrel in the areas between the insulating tubes and to adjacent portions of the insulating tubes and curing the liquid material to form the electrodes. This may be accomplished in stages, a relatively thin layer of liquid material being applied and cured prior to application of the next layer. Formation of the electrodes in this fashion avoids the need for careful fitting of preformed solid electrodes to the adjoining tubes to achieve a smooth exterior surface. Conversely, the electrodes or conductive tubes may be preformed and positioned on a mandrel and a curable liquid material may be applied so as to form insulating tubes having annular portions overlapping the exterior surfaces of the conductive tubes at the extremities of such tubes.

A probe according to another embodiment of the present invention is partially depicted in Fig. 8, this probe being similar to that described above with reference to Figs. 6 and 7. The probe illustrated in Fig. 8 incorporates a first insulating tube 210 formed primarily from a soft polymeric material, such polymeric material constituting the outermost layer of the tube defining its exterior surface 220. However, tube 210 has embedded in its wall a metallic conductor 222. An electrode 234 having an annular portion 242 terminating in a discrete sharp step 243 is mounted to tube 210 so that annular portion 242 surrounds the distal extremity of tube 210. An abutment wall 244 of the electrode confronts the distal end wall 246 of tube 210, and also confronts the distal end of the conductor 222. A layer of an electrically conductive bonding material 248 is disposed between electrode 234 and tube 210, such layer extending over the entire interface between these elements, including the interface between the electrode and the embedded conductor 222. A bead 250 formed from the bonding material extends between the exterior surface 252 of the electrode and the exterior surface 220 of tube 210 around the entire circumference of the probe. The bead covers the step 243 at the extremity of the electrode so that the electrode, bead and tube cooperatively define a smooth exterior surface. The conductive material of the electrode and the bonding material are both soft, primarily polymeric materials so that the electrode and bead are elastically continuous with the outermost portion of tube 210. However, these elements are not elastically continuous with conductor 222.

Electrode 234 and tube 210 may be joined with one another by juxtaposing the electrode with the tube and forming both a bead of the curable liquid phase at the extremity of the electrode and a portion of such curable liquid phase at the interface between the electrode and the tube. The curable liquid phase constituting the bead should extend between the exterior surfaces of the electrode and the tube so that it will flow, under the influence of its own surface and interfacial tensions to a smooth contour extending between such surfaces. The curable liquid phase can be formed at the desired location by applying a curable liquid material such as those described above by means of a brush, hypodermic needle or other applicator, either before or after juxtaposing the electrode and tube. Alternatively, such a curable liquid phase may be formed by selectively melting a portion of the electrode after juxtaposing these elements or immediately before juxtaposing them.

The use of a curable liquid bead at the extremity of the electrode permits ready assembly of preformed electrodes and tubes without the need of close fitting to form a smooth continuous surface on the exterior of the probe. The bead resulting from this process serves to reinforce the bond between the conductive tube or electrode and the insulating portion or tube at the extremity of the juncture therebetween. A similar technique can be applied whether or not the tube incorporates an embedded conductor. If the insulating tube does not incorporate an embedded conductor, the bonding material need not be electrically conductive. Thus, the liquid phase may be formed from a liquid material which does not incorporate any conductive material, and may be formed by melting portions of the insulating tube.

Numerous variations of the features described above may be utilized without departing from the present invention. Merely by way of example, the probes described above incorporate conductors which extend from the electrodes to the proximal end of the probe. However, if the probe has an interior bore of sufficient diameter to receive a separate electrical conductor, then the conductors incorporated in the probe itself may be arranged merely to conduct electrical signals between the electrodes and locations on the interior bore of the tube, such locations being accessible for connection with separately formed electrical conductors. The number of electrodes and the locations of the electrodes will vary according to the intended use of the probe. Thus, a probe for applying a stimulating voltage at a single location within the subject may incorporate only one electrode. Such a probe need not incorporate the additional sensing elements incorporated in the probes described above. Also, probes according to the present invention may be made in a wide variety of sizes and forms for insertion into various bodily passages of different human and animal subjects.

As these and other variations and combinations of the present invention may be utilized, the foregoing description of the preferred embodiments should be understood by way of illustration rather than by way of limitation of the present invention as set forth in the claims.

**Claims**

1. A biological probe for insertion into the body of a living organism, the probe comprising: an elongated, flexible sheath defining a smooth exterior surface (20), said sheath including an electrically insulating portion (10) composed of a soft polymeric material, said sheath also including a soft coating composed of a blend material including a polymeric material and an electrically conductive non-polymeric material, said coating overlying a surface of said insulating portion and constituting an electrode (34, 36), an exposed surface of said coating constituting a part of the exterior surface of the sheath, there being an electrical conductor (12, 14) composed of material including polymeric material and an electrically conductive non-polymeric material embedded in the insulating portion of the sheath, said insulating portion having a depression (22, 24) in its exterior surface extending inwardly to said conductor, said coating extending into said depression, and means for connecting said probe to a monitoring apparatus, characterized in that said coating adheres to the surface of said insulating portion and is fused with said conductor (12, 14) in said depression.

2. A probe as claimed in claim 1 in which said coating is composed of a soft material and the region of said insulating portion underlying said coating is also composed of a soft material, said coating and said underlying region being elastically continuous with one another.

3. A probe as claimed in claim 1 in which said conducting means includes an electrical conductor embedded in said insulating portion, said insulating portion having a depression in its surface extending inwardly to said conductor, said coating extending into said depression and contacting said conductor in said depression.

4. A probe as claimed in claim 3 in which said insulating portion is composed of a polymeric material, said conductor is composed of a blend material including a polymeric material and an electrically conductive material, said coating also being composed of a blend material including a polymeric material and an electrically conductive material, said coating being fused with said conductor in said depression.

5. A probe as claimed in claim 4 in which all of said polymeric materials consist primarily of polymers of a common monomer.

6. A probe as claimed in claim 5 in which said monomer is vinyl chloride.

7. A probe as claimed in any of the preceding claims in which said coating is no more than about 0.25 mm thick.

8. A method of forming a sheath for a biological probe including an electrically insulating portion and an electrically conductive portion constituting an electrode comprising the steps of:

(a) depositing a curable liquid material in juxtaposition with a solid body constituting one of said portions so that such liquid material wets the exterior surface of such solid and flows to a contour blending smoothly with such surface; and

(b) curing said liquid material to a solid form without disturbing said contour to form the other one of said portions.

9. A method as claimed in claim 8 in which said insulating portion is a solid, elongated sheath portion and said electrode is formed by depositing a coating of said curable liquid material on the exterior surface of said solid, elongated sheath portion.

10. A method as claimed in claim 9 further comprising the step of establishing electrical connection between the electrode and an electrical conductor embedded within said solid, elongated sheath portion by causing said liquid material to flow into contact with said conductor prior to completion of said curing step.

11. A method as claimed in claim 10 further comprising the steps of forming a depression in the exterior surface of said solid, elongated sheath portion and exposing a portion of the conductor in said depression before depositing the liquid material on such sheath portion, the liquid material contacting said conductor in said depression.

12. A method as claimed in claim 11 in which said solid, elongated sheath portion is a hollow tube, said depression being formed by removing a part of such wall without penetrating entirely through such wall, said liquid material being deposited so that it covers the margins of said depression.

13. A method as claimed in any of claims 10 through 12 in which said conductor is composed of a blend material including a polymer and a conductive material dispersed therein and said liquid material includes an electrically conductive material and a solvent for the polymer of said blend material.

14. A method as claimed in claim 13 in which the insulating material of said sheath portion includes a polymeric material and the liquid material incorporates a solvent for such polymeric material.

15. A method as claimed in claim 14 in which the liquid material includes a polymeric material, the polymeric material incorporated in said insulating material and the polymeric material incorporated in the liquid material both consisting primarily of polymers of vinyl chloride.

16. A method as claimed in any of claims 13 through 15 in which the electrically conductive material incorporated in said liquid material is carbon black.

17. A biological probe for insertion into the body of a living organism, the probe being an elongated, flexible member, having a proximal end, a distal end and comprising a sheath defining a smooth, exterior surface, said sheath including an electrically conductive portion constituting an electrode (134), and including a mass of conductive material having an exposed surface, said exposed surface of said conductive portion constituting a part of the exterior surface of the sheath, said sheath also including an electrically insulating portion (110), said mass being secured adjacent to the insulating portion in the axial direction, characterized in that, said mass is bonded to said insulating portion, such bond extending to the extremity (152) of the interface between said mass and said insulating portion, there being a smooth transition between the exposed surface of said mass and the exposed surface of said insulating portion.

18. A probe as claimed in claim 17 in which said insulating portion includes a first insulating tube and said mass of conductive material is in the form of a conductive tube, a proximal end of said conductive tube being connected to a distal end of said first insulating tube, axially-extensive portions of said tubes overlapping one another at the juncture therebetween, one such overlapping portion being disposed radially outboard of the other, the thickness of such radially outboard overlapping portion tapering towards the axial extremity of such portion, said bond extending between the confronting surfaces of said overlapping portions to the outer extremity of the interface between such confronting surfaces.

19. A probe as claimed in claim 18 further comprising a bead of bonding material at the outer extremity of said interface between the confronting surfaces of said overlapping portions, said bead extending between the exterior surfaces of said tubes and defining a smooth transition therebetween.

20. A probe as claimed in any of claims 17 through 19 in which said insulating portion and said conductive mass are both composed of soft materials, said insulating portion and said mass being elastically continuous with one another.

21. A method of making a biological probe comprising the steps of:

(a) juxtaposing a mass of an electrically conductive material with an electrically insulating sheath portion to form a composite sheath, and in a way to be adjacent in the axial direction of the composite sheath, an exposed surface of the conductive mass forming part of the exterior surface of the composite sheath, the interface between the conducting mass and the insulating portion extending to such exterior surface;

(b) forming a curable liquid phase at the interface between the conductive mass and the insulating portion, such liquid phase extending to the juncture of such interface and the exterior surface of the composite sheath, said liquid phase wetting both the conductive mass and the insulating portion; and

(c) curing said liquid phase to form a bond between the conductive mass and the insulating portion, such bond extending to the extremity of said interface at said exterior surface.

22. A method as claimed in claim 21 in which a bead of said liquid phase is formed prior to completion of said curing step, on the exterior surface of the composite sheath adjacent said interface, the liquid in said bead flowing to a smooth contour extending between the exposed surface of said mass and the adjacent exposed surface of said insulating portion, the liquid phase being cured without disturbing such smooth contour.

**Patentansprüche**

1. Biologische Sonde zum Einführen in den Körper eines lebenden Organismus, Sonde umfassend:

eine langgestreckte flexible Hülle, die eine glatte Außenfläche (20) definiert, welche Hülle einen elektrisch isolierenden Teil (10) aus einem weichen Polymer und außerdem einen weichen Überzug aus einem Mischwerkstoff enthält, der sich zusammensetzt aus einem Polymermaterial und einem elektrisch leitfähigen nicht-polymeren Material, welcher Überzug auf einer Oberfläche des isolierenden Teils liegt und eine Elektrode (34, 36) bildet, wobei eine freiliegende Fläche des Überzugs einen Teil der Außenseite der Hülle bildet, wobei ferner ein aus einem Werkstoff, der polymeres Material und ein elektrisch leitfähiges, nicht-polymeres Material enthält, bestehender elektrischer Leiter (12, 14) vorgesehen ist, der in den isolierenden Teil der Hülle eingebettet ist, wobei außerdem dieser isolierende Teil in seiner Außenseite eine Vertiefung (22, 24) aufweist, die nach innen in Richtung auf den Leiter zeigt, und wobei der Überzug in die Vertiefung hineinreicht, sowie eine Einrichtung zum Anschließen der Sonde an ein Kontrollgerät, dadurch gekennzeichnet, daß der Überzug an der Oberfläche des isolierenden Teils haftet und mit dem Leiter (12, 14) in der Vertiefung verschmolzen ist.

2. Sonde nach Anspruch 1, bei welcher der Überzug aus einem weichen Material besteht und der Bereich des unter dem Überzug liegenden isolierenden Teils ebenfalls aus einem weichen Material besteht, wobei der Überzug und der darunter liegende Bereich in Bezug aufeinander stetigelastisch sind.

3. Sonde nach Anspruch 1, bei welcher die leitende Einrichtung einen in dem isolierenden Teil eingebetteten elektrischen Leiter enthält, der isolierende Teil in seiner Oberfläche eine Vertiefung aufweist, die sich nach innen zu dem Leiter hin erstreckt, und der Überzug sich in die Vertiefung erstreckt und den Leiter in der Vertiefung kontaktbildend berührt.

4. Sonde nach Anspruch 3, bei welcher der isolierende Teil aus einem polymeren Material besteht, der Leiter aus einem Mischwerkstoff mit einem polymeren Material und einem elektrisch leitfähigen Material besteht, der Überzug ebenfalls aus einem Mischwerkstoff mit einem polymeren Material und einem elektrisch leitfähigen Material besteht, und der Überzug mit dem Leiter in der Vertiefung verschmolzen ist.

5. Sonde nach Anspruch 4, bei welcher sämtliche polymeren Materialien vorzugsweise aus Polymeren eines gemeinsamen Monomeren bestehen.

6. Sonde nach Anspruch 5, bei welcher das Monomer Vinylchlorid ist.

7. Sonde nach einem der vorhergehenden Ansprüche, bei welcher der Überzug nicht stärker als 0,25 mm ist.

8. Verfahren zum Herstellen einer Hülle für eine biologische Sone mit einem elektrisch isolierenden Teil und einem elektrisch leitfähigen Teil, der eine Elektrode darstellt, die folgenden Schritte umfassend:

(a) Aufbringen eines härtbaren flüssigen Materials in Berührung mit einem festen Körper, der einen der genannten Teile bildet, so daß das flüssige Material die Außenfläche des festen Körpers befeuchtet und zu einem Umriß verläuft, der glatt in diese Außenfläche übergeht, und

(b) Härten des flüssigen Materials zu einer festen Form ohne den Umriß zu stören, um den anderen dieser Teile zu bilden.

9. Verfahren nach Anspruch 8, bei welchem der isolierende Teil ein fester, langgestreckter Hüllenteil ist und die Elektrode dadurch gebildet wird, daß eine Schicht des härtbaren flüssigen Materials auf der Außenfläche des festen, langgestreckten Hüllenteils aufgebracht wird.

10. Verfahren nach Anspruch 9, ferner umfassend den Schritt, eine elektrische Verbindung zwischen der Elektrode und einem in den festen, langgestreckten Hüllenteil eingebetteten Leiter herzustellen, indem das flüssige Material veranlaßt wird, dem Leiter vor dem Härten kontaktbildend zuzufließen.

11. Verfahren nach Anspruch 10, ferner umfassend die Schritte, eine Vertiefung in der Außenfläche des festen, langgestreckten Hüllenteils zu bilden und einen Teil des Leiters in der Vertiefung freizulegen, bevor das flüssige Material auf den Hüllenteil aufgebracht wird, so daß das flüssige Material den Leiter in der Vertiefung kontaktbildend berührt.

12. Verfahren nach Anspruch 11, bei welchem der feste langgestreckte Hüllenteil als hohles Rohr ausgebildet ist, wobei die Vertiefung durch Entfernen eines Teilstücks dieser Wand gebildet wird, ohne die Wand vollständig zu durchstoßen, und wobei das flüssige Material so aufgebracht wird, daß es die Ränder der Vertiefung bedeckt.

13. Verfahren nach einem der Ansprüche 10 bis 12, bei welchem der Leiter aus einem Mischwerkstoff besteht, der ein Polymer und ein darin dispergiertes leitfähiges Material enthält, und das flüssige Material ein elektrisch leitfähiges Material und ein Lösungsmittel für das Polymer des Mischwerkstoffs enthält.

14. Verfahren nach Anspruch 13, bei welchem das isolierende Material des Hüllenteils ein Polymermaterial enthält und das flüssige Material ein Lösungsmittel für dieses Polymermaterial umfaßt.

15. Verfahren nach Anspruch 14, bei welchem das flüssige Material ein Polymermaterial enthält, wobei das in dem isolierenden Material enthaltene Polymermaterial sowie das in dem flüssigen Material enthaltene Polymermaterial vorzugsweise aus Polymeren von Vinylchlorid bestehen.

16. Verfahren nach einem der Ansprüche 13 bis 15, bei welchem das in dem flüssigen Material enthaltene elektrisch leitfähige Material Ruß ist.

17. Biologische Sonde zum Einführen in den Körper eines lebenden Organismus, wobei die Sonde ein langgestrecktes, flexibles Element mit einem proximalen und einem distalen Ende ist und eine Hülle aufweist, die eine glatte Außenfläche besitzt und einen elektrisch leitfähigen Teil enthält, der eine erste Elektrode (134) bildet, und ferner eine Masse von leitfähigem Material, die eine freiliegende Oberfläche besitzt, wobei die freiliegende Oberfläche des leitfähigen Teils ein Abschnitt der Außenfläche der Hülle ist und die Hülle außerdem einen elektrisch isolierenden Teil (110) aufweist, und wobei die Masse in axialer Richtung anstoßend an den isolierenden Teil in axialer Richtung festgelegt ist, dadurch gekennzeichnet, daß die Masse mit dem isolierenden Teil verbunden ist und das Bindemittel an das Ende (152) der Grenzfläche zwischen der Masse und dem isolierenden Teil reicht, wobei ein glatter Übergang zwischen der freiliegenden Fläche der Masse und der freiliegenden Fläche des isolierenden Teils besteht.

18. Sonde nach Anspruch 17, bei welcher der isolierende Teil ein erstes isolierendes Rohr aufweist und die Masse von leitendem Material die Form eines leitfähigen Rohrs hat, bei dem ein proximales Ende mit einem distalen Ende des ersten isolierenden Rohrs verbunden ist, wobei axial sich erstreckende Teile der Rohre an ihrer Verbindungsstelle einander überlappen und der eine der überlappenden Teile radial außerhalb des anderen liegt und die Stärke dieses radial außerhalb liegenden Teils sich in Richtung auf das axiale Ende dieses Teils verjüngt, und wobei das zwischen den einander gegenüberliegenden Flächen dieser einander überlappenden Teile befindliche Bindemittel sich bis zum äußeren Ende der Grenzfläche zwischen diesen einander gegenüberliegenden Flächen erstreckt.

19. Sonde nach Anspruch 18, weiter umfassend einen Wulst von Bindemittel am äußeren Ende der Grenzfläche zwischen den einander gegenüberliegenden Flächen der einander überlappenden Teile, wobei das Bindemittel sich zwischen den Außenflächen der Rohre erstreckt und zwischen diesen einen glatten Übergang herstellt.

20. Sonde nach einem der Ansprüche 17 bis 19, bei welcher sowohl der isolierende Teil als auch die leitfähige Masse aus weichem Material bestehen und der isolierende Teil und die Masse stetig-elastisch sind.

21. Verfahren zum Herstellen einer biologischen Sonde, die folgenden Schritte umfassend:

(a) eine Masse eines elektrisch leitfähigen Materials und eines elektrisch isolierenden Hüllenteils zur Bildung einer zusammengesetzten Hülle miteinander in einer Weise in Berührung bringen, daß sie in axialer Richtung der zusammengesetzten Hülle nebeneinanderliegen, wobei eine freiliegende Fläche der leitfähigen Masse einen Teil der Außenfläche der zusammengesetzten Hülle bildet und die Grenzfläche zwischen der leitfähigen Masse und dem isolierenden Teil bis an die Außenseite reicht;

(b) Herstellen einer härtbaren flüssigen Phase an der Grenzfläche zwischen der leitfähigen Masse und dem isolierenden Teil, wobei die flüssige Phase bis zum Zusammentreffen dieser Grenzfläche mit der Außenfläche der zusammengesetzten Hülle reicht und wobei die flüssige Phase sowohl die leitfähige Masse als auch den isolierenden Teil befeuchtet; und

(c) Härten der flüssigen Phase zur Bildung einer Verbindung zwischen der leitfähigen Masse und dem isolierenden Teil, wobei die Verbindung bis zu dem Ende der Grenzschicht an der Außenfläche reicht.

22. Verfahren nach Anspruch 21, bei welchem vor dem Abschluß des Härtungsschritts auf der Außenfläche der zusammengesetzten Hülle anstoßend an die Grenzfläche ein Wulst der flüssigen Phase gebildet wird, wobei die Flüssigkeit in dem Wulst sich zu einem glatten Umriß ausbreitet, der von der freiliegenden Fläche der Masse bis zu der anstoßenden freiliegenden Fläche des isolierenden Teils reicht, und wobei die flüssige Phase gehärtet wird, ohne diesen glatten Umriß zu stören.

**Revendications**

1. Sonde biologique destinée à être introduite dans le corps d'un organisme vivant, la sonde comprenant:

une gaine souple allongée délimitant une surface externe lisse (20), la gaine contenant une partie isolante de l'électricité (10) composée d'un matériau polymère mou, la gaine contenant aussi un revêtement mou composé d'un matériau mélangé contenant un matériau polymère et un matériau non polymère conducteur de l'électricité, le revêtement recouvrant une surface de la partie isolante et constituant une électrode (34, 36), une surface exposée du revêtement constituant une partie de la surface externe de la gaine, un conducteur électrique (12, 14) composé d'un matériau contenant un matériau polymère et un matériau non polymère et conducteur de l'électricité, étant enrobé dans la partie isolante de la gaine, cette partie isolante ayant une cavité (22, 24) à sa surface externe et tournée vers l'intérieur du conducteur, le revêtement pénétrant dans la cavité, et un dispositif de raccordement de la sonde à un appareil de contrôle, caractérisée en ce que le revêtement adhère à la surface de la partie isolante et est associé par fusion audit conducteur (12, 14) dans la cavité.

2. Sonde selon la revendication 1, dans laquelle le revêtement est composé d'un matériau mou et la région de la partie isolante placée au-dessous du revêtement est aussi composée d'un matériau mou, le revêtement et la région sous-jacente présentant une continuité élastique l'un avec l'autre.

3. Sonde selon la revendication 1, dans laquelle le dispositif conducteur comprend un conducteur électrique enrobé dans la partie isolante, cette partie isolante ayant une cavité à sa surface, la cavité étant tournée vers l'intérieur jusqu'au conducteur, le revêtement pénétrant dans la cavité et étant au contact du conducteur placé dans la cavité.

4. Sonde selon la revendication 3, dans laquelle la partie isolante est composée d'un matériau polymère, le conducteur est composé d'un matériau mélangé contenant un matériau polymère et un matériau conducteur de l'électricité, le revêtement étant aussi composé d'un matériau mélangé contenant un matériau polymère et un matériau conducteur de l'électricité, le revêtement étant associé par fusion au conducteur dans la cavité.

5. Sonde selon la revendication 4, dans laquelle tous les matériaux polymères sont essentiellement formés de polymères d'un monomère commun.

6. Sonde selon la revendication 5, dans laquelle le monomère est le chlorure de vinyle.

7. Sonde selon l'une quelconque des revendications précédentes, dans laquelle le revêtement a une épaisseur qui ne dépasse pas 0,25 mm environ.

8. Procédé de formation d'une gaine destinée à une sonde biologique, comprenant une partie isolante de l'électricité et une partie conductrice de l'électricité, constituant une électrode, comprenant les étapes suivantes:

(a) le dépôt d'un matériau liquide durcissable juxtaposé à un corps solide constituant l'une desdites parties afin que ce matériau liquide mouille la surface externe du corps solide et s'écoule en prenant un profil se raccordant progressivement à ladite surface, et

(b) le durcissement du matériau liquide sous forme solide sans perturbation du profil afin que l'autre desdites parties soit formée.

9. Procédé selon la revendication 8, dans lequel la partie isolante est une partie allongée et solide de gaine et l'électrode est formée par dépôt d'un revêtement du matériau liquide durcissable à la surface externe de la partie solide allongée de gaine.

10. Procédé selon la revendication 9, comprenant l'étape d'établissement d'une connexion électrique entre l'électrode et un conducteur électrique enrobé dans la partie solide et allongée de gaine, par écoulement du matériau liquide au contact du conducteur avant la fin de l'étape de durcissement.

11. Procédé selon la revendication 10, comprenant en outre des étapes de formation d'une cavité à la surface externe de la partie solidaire allongée de gaine et d'exposition d'une partie du conducteur dans la cavité avant dépôt du matériau liquide sur la partie de gaine, le matériau liquide étant au contact du conducteur placé dans la cavité.

12. Procédé selon la revendication 11, dans lequel la partie solide et allongée de gaine est un tube, la cavité étant formée par enlèvement d'une partie de la paroi sans traversée de celle-ci, le matériau liquide étant déposé de manière qu'il recouvre les marges de la cavité.

13. Procédé selon l'une quelconque des revendications 10 à 12, dans lequel le conducteur est composé d'un matériau mélangé contenant un polymère et d'un matériau conducteur dispersé à l'intérieur, et le matériau liquide contient un matériau conducteur de l'électricité et un solvant du polymère du matériau mélangé.

14. Procédé selon la revendication 13, dans lequel le matériau isolant de la partie de gaine contient un matériau polymère et le matériau liquide contient un solvant d'un matériau polymère.

15. Procédé selon la revendication 14, dans lequel le matériau liquide contient un matériau polymère, le matériau polymère incorporé au matériau isolant et le matériau polymère incorporé au matériau liquide consistant tous deux essentiellement en polymères de chlorure de vinyle.

16. Procédé selon l'une quelconque des revendications 13 à 15, dans lequel le matériau conducteur de l'électricité incorporé au matériau liquide est du noir de carbone.

17. Sonde biologique destinée à être introduite dans le corps d'un organisme vivant, la sonde étant un organe souple et allongé ayant une extrémité proche, une extrémité éloignée et comprenant une gaine délimitant une surface externe lisse, la gaine comprenant une partie conductrice de l'électricité constituant une électrode (134) et contenant une masse d'un matériau conducteur ayant une surface exposée, la surface exposée de la partie conductrice constituant une partie de la surface externe de la gaine, la gaine contenant aussi une partie (110) isolante de l'électricité, la masse étant fixée près de la partie isolante, dans la direction axiale, caractérisée en ce que la masse est liée à la partie isolante, la liaison rejoignant l'extrémité (152) de l'interface de la masse et de la partie isolante, une transition régulière existant entre la surface exposée de la masse et la surface exposée de la partie isolante.

18. Sonde selon la revendication 17, dans laquelle la partie isolante contient un premier tube isolant et la masse du matériau conducteur est sous forme d'un tube conducteur, une extrémité proche du tube conducteur étant raccordée à une extrémité éloignée du premier tube isolant, des parties des tubes disposées axialement se recouvrant à leurs jonctions, l'une des parties en recouvrement étant disposée radialement vers l'extérieur par rapport à l'autre, l'épaisseur de la partie radialement externe en recouvrement diminuant progressivement vers l'extrémité axiale de cette partie, la liaison rejoignant les surfaces en regard des parties en recouvrement vers l'extrémité externe de l'interface de ces surfaces en regard.

19. Sonde selon la revendication 18, comprenant en outre un cordon d'un matériau de liaison à l'extrémité externe de l'interface, entre les surfaces en regard des parties en recouvrement, le cordon étant disposé entre les surfaces externes des tubes et délimitant une transition lisse entre eux.

20. Sonde selon l'une quelconque des revendications 17 à 19, dans laquelle la partie isolante et la masse conductrice sont toutes deux composées de matériaux mous, la partie isolante et la masse présentant une continuité élastique l'une avec l'autre.

21. Procédé de fabrication d'une sonde biologique, comprenant les étapes suivantes:

(a) la juxtaposition d'une masse d'un matériau conducteur de l'électricité et d'une partie de gaine isolante de l'électricité afin qu'une gaine composite soit formée, d'une manière telle qu'elle est adjacente dans la direction axiale de la gaine composite, une surface exposée de la masse conductrice formane une partie de la surface externe de la gaine composite, l'interface de la masse conductrice et de la partie isolante rejoignant la surface externe,

(b) la formation d'une phase liquide durcissable à l'interface de la masse conductrice et de la partie isolante, la phase liquide rejoignant la jonction de l'interface et la surface externe de la gaine composite, la phase liquide mouillant à la fois la masse conductrice et la partie isolante, et

(c) le durcissement de la phase liquide afin qu'elle forme une liaison entre la masse conductrice et la partie isolante, la liaison rejoignant l'extrémité de l'interface à la surface externe.

22. Procédé selon la revendication 21, dans lequel un cordon de la phase liquide est formé avant la fin de l'étape de durcissement, à la surface externe de la gaine composite, près de l'interface, le liquide du cordon s'écoulant en prenant un profil lisse disposé entre la surface exposée de la masse et la surface exposée adjacente de la partie isolante, la phase liquide étant durcie sans perturbation du profil lisse.

0 101 595

Fig. 1.

Fig. 2.

Fig. 3.

## Fig. 4.

COEXTRUDED TUBE → | EXPOSE CONDUCTORS | → | APPLY LIQUID | → | CURE | → TO FINAL ASSEMBLY

## Fig. 5.

Fig.6.

Fig.7.

Fig.8.

0 101 595